# FASCICULE DE BREVET EUROPEEN

(11) **EP 0 635 258 B1**
(45) Date de publication et mention de la délivrance du brevet: **15.05.2002**
(21) Numéro de dépôt: 94401647.6
(22) Date de dépôt: 19.07.1994
(51) Int. Cl.: A61K 7/06, A61K 7/00, A61K 7/48

(54) **Compositions cosmétiques pour le maintien de la coiffure présentant un pouvoir fixant amélioré**
Kosmetische Zusammensetzungen für den Halt der Frisur mit verbessertem Fixiervermögen
Cosmetic compositions with improved fixing power

(30) Priorité: 23.07.1993 FR 9309096
(43) Date de publication de la demande: 25.01.1995
(73) Titulaire: L'OREAL, 75008 Paris (FR)
(72) Inventeur: Dupuis, Christine, F-75018 Paris (FR)
(74) Mandataire: Andral, Christophe André Louis

(56) Documents cités:
- EP-A- 0 492 657
- EP-A- 0 535 367
- GB-A- 2 034 724

## Description

La présente invention concerne l'amélioration du pouvoir fixant des compositions cosmétiques pour le maintien de la coiffure contenant un polymère anionique.

Dans le cadre de la présente demande on entendra par compositions cosmétiques pour le maintien de la coiffure toute composition ayant pour objet de fixer temporairement la forme de la coiffure comme par exemple les laques, les compositions de mise en plis, les mousses, les gels et sprays de coiffage. Par pouvoir fixant de la composition on désignera l'aptitude de cette dernière à donner aux cheveux une cohésion telle que la mise en forme initiale de la coiffure est conservée.

La mise en oeuvre de dérivés siliconés en association avec des résines polymères est largement connue dans la préparation de compositions cosmétiques pour le maintien de la coiffure. Il a été constaté que ces dérivés siliconés améliorent de façon nette les propriétés de démêlage, de douceur et de brillance des cheveux traités à l'aide de ces compositions. Inversement, ils ne sont pas favorables au pouvoir fixant de ces dernières, assuré par la résine polymère.

On connait aussi dans l'art antérieur, la demande EP 492 657 A1 qui décrit la mise en oeuvre de copolymères blocs linéaires polysiloxane-polyoxyalkylène dans des compositions cosmétiques utiles pour le soin de la peau et le soin des cheveux. Selon cette demande, l'utilisation de ces copolymères seuls ou avec un agent de traitement de la peau ou des cheveux, permet d'obtenir des propriétés améliorées de brillance, de durabilité et/ou de douceur de la formulation. Ces copolymères sont décrits de façon générale comme présentant, par rapport aux composés siliconés de l'art antérieur utilisés dans ces compositions, des propriétés de même nature mais d'un degré sensiblement plus élevé.

L'homme de l'art doit donc s'attendre à ce que les copolymères décrits dans la demande de brevet européen précitée aggrave encore la détérioration du pouvoir fixant, même si par ailleurs il s'attend à une amélioration des caractéristiques de démêlage, de brillance et de douceur. Ce point n'est pas démenti par la lecture des exemples de cette demande notamment par l'exemple 19 dont la reproduction conduit à une composition ayant un pouvoir fixant très faible incompatible avec une commercialisation de cette dernière.

Il est décrit dans ce même document que les copolymères peuvent être utilisés conjointement à des additifs dont des composés à haut poids moléculaire comme notamment les trois polymères suivants : copolymère anhydride maléique / méthyl vinyl ether monoestérifié par un alcool inférieur, copolymère acétate de vinyle / acide crotonique et copolymère acide acrylique / ester acrylique / N-alkyl acrylamide. Ces trois composés ne sont en rien distingués en tant que polymères anioniques apportant des propriétés particulières.

Il n'est de plus nulle part fait mention dans la demande européenne précitée de l'intérêt spécifique que pourraient présenter ces copolymères blocs linéaires pour obtenir des compositions de maintien de la coiffure présentant un pouvoir fixant amélioré.

La demanderesse a découvert qu'en associant les copolymères blocs polysiloxane-polyoxyalkylène linéaires aux résines polymères anioniques dans les compositions pour le maintien de la coiffure, on obtient de façon surprenante des compositions conférant de bonnes caractéristiques de démêlage, de brillance et de douceur aux cheveux tout en apportant un pouvoir fixant amélioré.

L'invention a donc pour objet l'utilisation d'un copolymère bloc linéaire polysiloxane-polyoxyalkylène comme agent renforçateur du pouvoir fixant d'une composition pour le maintien de la coiffure contenant un polymère anionique.

L'invention concerne également une nouvelle composition cosmétique pour la mise en oeuvre du procédé ci-dessus caractérisée en ce qu'elle contient dans un support cosmétiquement acceptable un copolymère bloc linéaire polysiloxane-polyoxyalkylène et un polymère anionique à l'exclusion des polymères anioniques suivants : copolymère anhydride maléique / méthyl vinyl ether monoestérifié par un alcool inférieur, copolymère acétate de vinyle / acide crotonique et copolymère acide acrylique / ester acrylique / N-alkyl acrylamide.

Selon un mode de réalisation particulier de l'invention le polymère anionique est choisi parmi le groupe des polymères anioniques à groupement sulfonique comprenant les sels de l'acide polystyrène sulfonique, les sels de métaux alcalins et alcalino terreux des acides sulfoniques dérivés de la lignine, les sels de polyacrylamide sulfoniques, les polymères contenant des motifs alkylnaphtalène sulfonique salifiés, les polymères contenant un motif vinylsulfonique, les copolymères résultant de la polymérisation d'un acide sulfonique insaturé et d'un N-monoalkylacrylamide.

On peut citer comme exemples de sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.

On peut citer comme exemples de sels de métaux alcalins et alcalino terreux des acides sulfoniques dérivés de la lignine, les lignosulfonates de calcium ou de sodium tels que le produit vendu sous la dénomination Marasperse C-21 par American Can Co. et ceux en C₁₀-C₁₄ vendus par Avébène.

On peut citer comme exemples de sels de polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

On peut citer comme exemple de polymère contenant des motifs acide alkylnaphtalène sulfonique salifié, le sel de sodium vendu sous la dénomination Darvan n° 1 par Van der Bilt.

On peut citer comme exemples de polymères comportant dans leur chaîne au moins un motif vinylsulfonique, les polyvinylsulfonates ayant un poids moléculaire compris entre 1000 et 100.000 et notamment leurs sels de sodium, de potassium, de calcium, d'ammonium et les sels d'amines comme les sels d'alkylamines, d'alcanolamines ainsi que les copolymères comportant au moins des groupements vinylsulfoniques avec un ou plusieurs comonomères cosmétiquement acceptables tels que des acides insaturés choisis parmi les acides acrylique, méthacrylique et leurs esters, les amides tels que l'acrylamide ou le méthacrylamide substitués ou non, les esters vinyliques, les éthers vinyliques et la vinylpyrrolidone. Ces polymères sont décrits plus particulièrement dans les brevets français 2.238.474 et américain 2.961.431 et 4.138.477.

On peut citer comme exemples de copolymères résultant de la polymèrisation d'un acide sulfonique insaturé et d'un N-monoalkylacrylamide les polymères issus de la polymérisation d'au moins un acide sulfonique insaturé en une proportion de 30 à 90 % en poids et ayant la formule générale suivante : 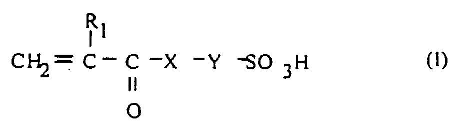 dans laquelle :
- R₁ représente un atome d'hydrogène ou le radical -CH₃,
- X représente -O- ou -NH-,
- Y représente une chaîne alkylène, linéaire ou ramifiée, ayant de 1 à 6 atomes de carbone, et d'au moins un N-monoalkylacrylamide ou méthacrylamide en une proportion de 10 à 70 % en poids et ayant la formule générale suivante : 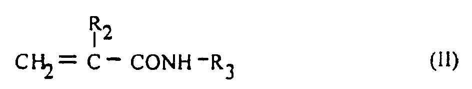 dans laquelle :
   - R₂ représente un atome d'hydrogène ou le radical -CH₃,
   - R₃ représente un radical alkyle, linéaire ou ramifié, ayant de 3 à 10 atomes de carbone,
les fonctions acides sulfoniques dudit polymère étant neutralisées en une proportion de 40 à 70 % par de la triéthanolamine.

Parmi les acides sulfoniques insaturés de formule générale (I), on peut citer notamment l'acide acrylamido-2 méthyl-2 propane sulfonique, le méthacrylate de 2-sulfoéthyle, la N-acryloyltaurine et la N-méthacryloyltaurine.

Parmi les N-monoalkylacrylamides ou méthacrylamides de formule générale (II), on peut citer notamment le N-tertiobutylacrylamide, le N-tertiohexylacrylamide et le N-tertiooctylacrylamide.

Encore plus préférentiellement, on peut citer le produit de la copolymérisation d'un acide amidosulfonique (X=-NH-) dont la chaîne alkylène a de 2 à 4 atomes de carbone, et en particulier, de l'acide acrylamido-2 méthyl-2 propane sulfonique. en une proportion de 40 à 70 % en poids et d'un N-monoalkylacrylamide et en particulier la N-tertiobutylacrylamide.

Ces copolymères peuvent également se présenter sous forme de ter- ou tétra-polymères.

Selon cette forme de réalisation, les comonomères susceptibles de constituer les autres unités répétitives du copolymère, peuvent être choisis parmi :
1) les acrylates ou méthacrylates d'alkyle en une proportion de 3 à 40 % en poids et ayant la formule générale suivante : 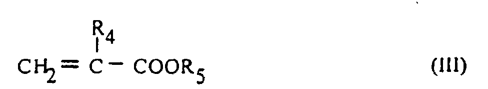 dans laquelle :
   - R₄ représente un atome d'hydrogène ou un radical -CH₃,
   - R₅ représente un radical alkyle, linéaire ou ramifié, ayant de 1 à 4 atomes de carbone.

   Parmi les acrylates et méthacrylates d'alkyle de formule (III), on peut citer l'acrylate de méthyle, l'acrylate d'éthyle, et le méthacrylate de butyle.
   Selon une forme de réalisation particulière, on préfère utiliser l'acrylate de méthyle ou l'acrylate d'éthyle en une proportion de 3 à 25 % en poids.
2) les acrylamides et méthacrylamides en une proportion de 3 à 40 % en poids et ayant la formule générale suivante : 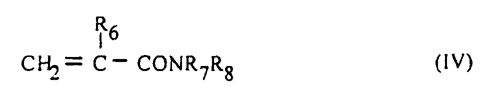 dans laquelle
   - R₆ représente un atome d'hydrogène ou le radical -CH₃,
   - R₇ et R₈, identiques ou différents, représentent un atome d'hydrogène ou un radical alkyle ayant de 1 à 4 atomes de carbone, ou R₇ représente un atome d'hydrogène et R₈ représente le radical :

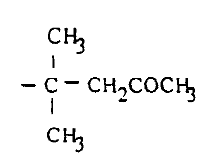

Parmi les acrylamides et méthacrylamides de formule (IV), on peut citer notamment le diméthyl-oxo-3-butyl-acrylamide, le N,N-diméthylacrylamide et le N, N-diéthylacrylamide. On préfère utiliser le diméthyl-oxo-3-butyl-acrylamide en une proportion de 3 à 25 %.

Parmi les copolymères encore plus particulièrement préférés, on peut notamment mentionner ceux comportant des unités répétitives dérivant de la copolymérisation des composés suivants :
- acide acrylamido-2 méthyl-2 propane sulfonique (62 %)/N-tertiobutylacrylamide (38 %),
- acide acrylamido-2 méthyl-2 propane sulfonique (40 %)/N-tertiobutylacrylamide (20 %)/acrylate d'éthyle (15 %)/diméthyl-oxo-3-butylacrylamide (25 %),
- acide acrylamido-2 méthyl-2 propane sulfonique (60 %)/N-tertiobutylacrylamide (20 %)/diméthyl-oxo-3-butylacrylamide (20 %),
- acide acrylamido-2 méthyl-2 propane sulfonique (60 %)/N-tertiobutylacrylamide (25 %)/acrylate d'éthyle (15 %), et
- acide acrylamido-2 méthyl-2 propane sulfonique (60 %)/N-tertiobutylacrylamide (25 %)/acrylate de méthyle (15 %).

Selon un autre mode de réalisation particulier de l'invention, le polymère anionique est un polymère à fonction acide carboxylique tel que décrit dans FR 2439798 et ayant la formule générale suivante : 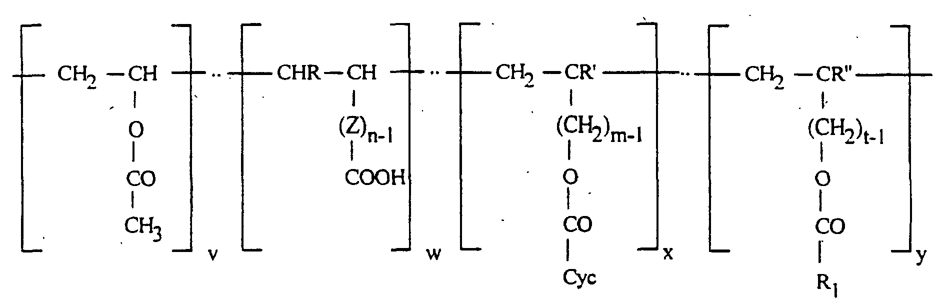 dans laquelle :
- R, R' et R", identiques ou différents, représentent un atome d'hydrogène ou un radical méthyle,
- m, n et t sont 1 ou 2,
- R₁ représente un radical alkyle, linéaire ou ramifié, saturé ou insaturé ayant de 2 à 21 atomes de carbone,
- Z représente un radical divalent pris dans le groupe constitué par : -CH₂-, -CH₂-O-CH₂- et -CH₂-O-(CH₂)₂-,
- Cyc représente un radical choisi parmi :
   (i) un radical de formule : 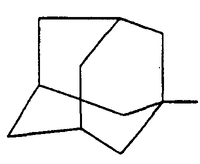
   (ii) un radical de formule 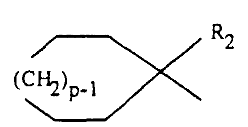 dans laquelle :
      R₂ représente un atome d'hydrogène ou un radical méthyle, et p est 1 ou 2.
   (iii) un radical de formule : 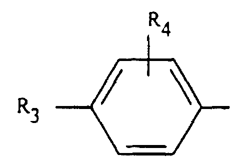 dans laquelle :
      - R₃ représente un atome d'hydrogène, un radical méthyle, éthyle, t-butyle, éthoxy, butoxy ou dodécyloxy,
      - R₄ représente un atome d'hydrogène, un radical alkyle de 1 à 4 atomes de carbone ou un radical alkoxy de 1 à 4 atomes de carbone,
   (iv) un radical de formule : 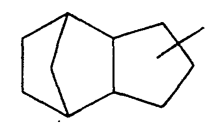
      - v représente de 10 à 91 % et de préférence de 36 à 84 % en poids.
      - w représente de 3 à 20 % et de préférence de 6 à 12 % en poids,
      - x représente de 4 à 60 % et de préférence de 6 à 40 % en poids.
      - y représente de 0 à 40 % et de préférence de 4 à 30 % en poids,
      - v + w + x + y étant égal à 100 %.

Parmi ces composés, on préfère plus particulièrement utiliser un terpolymère acétate de vinyle / tertio-butylbenzoate de vinyle / acide crotonique.

Selon un autre mode de réalisation particulier le polymère anionique est un copolymère vinylpyrrolidone / acide (méth)acrylique comprenant éventuellement d'autres comonomères tels que les acrylates ou méthacrylates d'alkyle.

On peut citer comme exemples les copolymères suivants:
- vinylpyrrolidone / acide acrylique comme les composés vendus sous les dénominations ACRYLIDONE ACP 1033, 1001, 1042 par I.S.P.,
- vinylpyrrolidone / acide méthacrylique / acrylate de t-butyle vendu sous les appellations LUVIFLEX VBM 35 et LUVIFLEX VBM 70 par BASF.
- vinylpyrrolidone / acide acrylique / méthacrylate de lauryle vendu sous la dénomination ACP-1135 par I.S.P.
Selon un autre mode de réalisation particulier de l'invention le polymère anionique est un copolymère acide (méth)acrylique/(méth)acrylate d'alkyle.

Selon un autre mode de réalisation particulier de l'invention le polymère anionique est un copolymère acide acrylique/N.N-diméthylacrylamide /- méthacrylate d'éthyle/N-tertiobutylacrylamide.

Les polymères anioniques utilisés dans le cadre de la présente invention peuvent se présenter dans les compositions finales soit dans un état solubilisé soit encore dans un état dispersé, par exemple sous la forme de latex et/ou de pseudolatex.

Les copolymères blocs linéaires polysiloxane-polyoxyalkylène utilisés dans le cadre de la présente invention ont de préférence la formule générale suivante :

([Y(R₂SiO)ₐR'₂SiYO][(CₙH₂ₙO)_{b}])_{c}

dans laquelle :
- R et R' identiques ou différents, représentent un radical hydrocarboné monovalent ne contenant pas d'insaturation aliphatique,
- n est un nombre entier compris entre 2 et 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 90% environ en poids du copolymère bloc,
- le poids moléculaire moyen du copolymère bloc étant d'au moins 3.000.

R et R' sont plus préférentiellement choisis parmi le groupe comprenant les radicaux alkyle comme par exemple les radicaux méthyle, ethyle, propyle, butyle, pentyle, hexyle, octyle, decyle, dodecyle, les radicaux aryle comme par exemple phényle, naphtyle, les radicaux aralkyle comme par exemple benzyle, phényléthyle, les radicaux tolyle, xylyle et cyclohexyle.

Y est de préférence -R"-, -R"-CO-, -R"-NHCO-, -R"-NH-CO-NH-R"'-NHCO. -R"-OCONH-R"'-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄-, -C₆H₄-CH(CH₃)₂-C₆H₄-.

Encore plus préférentiellement Y représente un radical alkylène divalent, plus particulièrement le radical -CH₂-CH₂-CH₂-.

La préparation des copolymères blocs mis en oeuvre dans le cadre de la présente invention est décrite dans la demande européenne EP 0 492 657 Al.

Les copolymères blocs linéaires polysiloxane-polyoxyalkylène préférés selon l'invention sont choisis parmi ceux de formule:

[C₄H₈ (CₙH₂ₙO)_{b}-C₄H₈-SiMe₂O(SiMe₂O)ₐSiMe₂]_{c}

ou Me représente méthyle, n est un entier de 2 à 4, a et b sont des entiers supérieurs ou égaux à 4, c est un nombre supérieur ou égal à 4.

Selon un mode de réalisation particulier de l'invention le copolymère bloc est choisi parmi les copolymères suivants :
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

Le polymère anionique est de préférence mis en oeuvre en une quantité comprise entre 0,1 et 25% en poids du poids total de la composition. Encore plus préférentiellement, cette quantité est comprise entre 0,5 et 20%.

En ce qui concerne le copolymère bloc linéaire il est utilisé de préférence en une quantité comprise entre 0,05 et 20% en poids du poids total de la composition. Encore plus préférentiellement, cette quantité est comprise entre 0,1 et 10%.

Les compositions selon l'invention peuvent se présenter sous forme de gel. d'émulsion (lait ou crème), de lotion aqueuse alcoolique ou hydroalcoolique plus ou moins épaissie de dispersion ou de mousse.

Elles sont plus particulièrement des lotions de mise en plis. des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple. lorsqu'on souhaite obtenir un spray, une laque ou une mousse pour la fixation des cheveux.

Le milieu cosmétiquement acceptable est par exemple constitué par de l'eau, un solvant organique comme par exemple un alcool et leurs mélanges.

Les compositions selon l'invention peuvent en outre contenir les additifs habituellement utilisés en cosmétique.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane et leurs mélanges, éventuellement avec au moins un hydrocarbure chloré et/ou fluoré.On peut également utiliser en tant qu'agent propulseur le gaz carbonique, le protoxyde d'azote, le diméthylether, l'azote ou l'air comprimé.

L'invention va être maintenant plus complètement illustrée à l'aide des exemples suivants qui ne sauraient être considérés comme la limitant aux modes de réalisation décrits.

### EXEMPLE 1

On prépare une laque aérosol en mélangeant les constituants suivants :
- 10 g. (matière active) du copolymère acétate de vinyle (65%)/acide crotonique (10%)(10%)/tertiobutylbenzoate de vinyle (25%),
- la quantité nécessaire de amino-2 méthyl-2 propanol-1 pour neutraliser 100% des fonctions acide du copolymère.
- 3g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- éthanol qsp 100g.

Cette composition est conditionnée dans un bidon aérosol selon le schéma de pressurisation suivant:
- Composition ci-dessus : 40%
- Diméthyléther: 40%
- Pentane : 20%

### EXEMPLE 2

On prépare un spray aérosol en mélangeant les constituants suivants :
- 8 g. (matière active) du copolymère acétate de vinyle (65%)/acide crotonique (10%)/tertiobutylbenzoate de vinyle (25%),
- la quantité nécessaire de amino-2 méthyl-2 propanol-1 pour neutraliser 100% des fonctions acide du copolymère,
- 2g de copolymère bloc de formule

   [[(CH₃]₂SiO]₉(CH₃)₂SiCH₂CH(CH₃)CH₂-O(C₂H₄O)₂₀-(C₃H₆O)₂₉CH₂CH(CH₃)CH₂]_{26.3}
- 10g. d'éthanol
- eau qsp 100g.

Cette composition est conditionnée selon le schéma de pressurisation suivant :
- composition ci-dessus: 70%
- diméthyléther: 30%.

### EXEMPLE 3

On prépare un spray pour flacon pompe en mélangeant les constituants suivants :
- 8 g. (matière active) du copolymère acétate de vinyle (65%)/acide crotonique (10%)/tertiobutylbenzoate de vinyle (25%),
- la quantité nécessaire de amino-2 méthyl-2 propanol-1 pour neutraliser 100% des fonctions acide du copolymère,
- 1,5g de copolymère bloc de formule

   [[[CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- 15g. d'eau
- éthanol qsp 100g.

### EXEMPLE 4

On prépare une lotion de mise en forme en mélangeant les constituants suivants :
- 0,5 g (matière active) d'acide polyacrylamidoéthylpropane sulfonique (vendu sous la dénomination COSMEDIA POLYMER HSP 1180 par Henkel,
- 1g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- 8,3 g. d'éthanol
- parfums, conservateurs qs,
- Eau qsp 100 g.

### EXEMPLE 5

On prépare une laque aérosol en mélangeant les constituants suivants :
- 8 g. (matière active) du terpolymère vinylpyrrolidone / acide méthacrylique /- acrylate de t-bentyle vendu sous la dénomination LUVIFLEX VBM 35 par BASF
- la quantité nécessaire de amino-2 méthyl-2 propanol-1 pour neutraliser à 100 % le LUVIFLEX VBM 35
- 2 g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- Ethanol absolu qsp 100 g

Cette composition est conditionnée selon le schéma de pressurisation suivant :
- composition ci-dessus : 37 g %
- diméthylether: 43 g %
- N pentane : 20 g %

### EXEMPLE 6

On prépare une mousse aérosol en mélangeant les constituants suivants :
- 1,8 g (matière active) du copolymère acide acrylamido-2 méthyl-2 propane sulfonique (62 %) / N-tertiobutylacrylamide (38 %)
- La quantité nécessaire de triéthanolamide pour neutraliser à 100 % le copolymère sulfonique
- 2 g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- 20 g d'éthanol absolu
- Eau qsp 100 g

Cette composition est conditionnée selon le schéma de pressurisation suivant :
- composition ci-dessus : 90 g %
- mélange butane / propane / isobutane vendu sous la dénomination AEROGAZ 3.2 N par ELF AQUITAINE : 10 g %

### EXEMPLE 7

On prépare un gel en mélangeant les constituants suivants :
- 2 g. (matière active) du copolymère acide méthacrylique (50 %) / méthacrylate de méthyle (50 %)
- la quantité nécessaire de amino-2 méthyl-2 propanol-1 pour neutraliser à 100 % le copolymère anionique
- 1 g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- 2 g. (matière active) d'épaississant acrylique vendu sous la dénomination CARBOPOL 940 par la société GOODRICH
- 40 g d'éthanol absolu
- la quantité nécessaire de amino-2 méthyl-2 propanol-1 pour neutraliser 100 % des fonctions acides du copolymère pH = 7,6
- Eau qsp 100 g

### EXEMPLE 8

On prépare un spray en mélangeant les constituants suivants :
- 8 g. (matière active) du copolymère acide méthacrylique (50 %) / méthacrylate de méthyle (50 %)
- la quantité nécessaire de amino-2 méthyl-2 propanol-1 pour neutraliser 100 % du copolymère anionique
- 2 g de copolymère bloc de formule

   [[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
- Ethanol absolu qsp 100 g

L'actif est conditionné dans un flacon pompe.

## Revendications

1. Utilisation d'un copolymère bloc linéaire polysiloxane-polyoxyalkylène comme agent renforçateur du pouvoir fixant d'une composition pour le maintien de la coiffure contenant un polymère anionique.

2. Utilisation selon la revendication 1 **caractérisée en ce que** le copolymère bloc linéaire répond à la formule générale
([Y(R₂SiO)ₐR'₂SiYO][(CₙH₂ₙO)_{b}])_{c}
dans laquelle
- R et R' identiques ou différents, représentent un radical hydrocarboné monovalent ne contenant pas d'insaturation aliphatique,
- n est un nombre entier compris entre 2 et 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 90% environ en poids du copolymère bloc,
- le poids moléculaire moyen du copolymère bloc étant d'au moins 3.000.

3. Utilisation selon la revendication 2 **caractérisée en ce que** R et R' sont choisis parmi le groupe comprenant les radicaux méthyle, ethyle, propyle. butyle, pentyle, hexyle, octyle, decyle, dodecyle, phényle, naphtyle, benzyle, phénylethyle. tolyle, xylyle et cyclohexyle.

4. Utilisation selon l'une quelconque des revendications 2 et 3 **caractérisée en ce que** Y est choisi parmi le groupe comprenant -R"-, -R"-CO-, -R"-NHCO-, R"-NH-CONH-R"'NHCO, -R"-OCONH-R"'-NHCO-, où R" représente un radical éthylène, propylène ou butylène et R"' représente un groupe -C₆H₄-, -C₆H₄-C₆H₄-, -C₆H₄-CH₂-C₆H₄-, ou -C₆H₄-C(CH₃)₂-C₆H₄-.

5. Utilisation selon l'une quelconque des revendications précédentes. **caractérisée en ce que** le copolymère bloc est choisi parmi ceux de formule:
[C₄H₈(CₙH₂ₙO)_{b}-C₄H₈-SiMe₂O(SiMe₂O)ₐSiMe₂]_{c}
ou Me représente méthyle , n est un entier de 2 à 4, a et b sont des entiers supérieurs ou égaux à 4, c est un nombre supérieur ou égal à 4.

6. Utilisation selon l'une quelconque des revendications précédentes **caractérisé en ce que** le copolymère bloc est choisi parmi le groupe comprenant les composés suivants :
[[(CH₃)₂SiO]₄₁(CH₃)₂SiCH₂CH(CH₃)CH₂-O(C₂H₄O)₁₈-(C₃H₆O)₃₃CH₂CH(CH₃)CH₂]_{16.1}
[[(CH₃)₂SiO)₃₁(CH₃)₂SiCH₂CH(CH₃)CH₂-O(C₂H₄O)₂₀-(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

7. Utilisation suivant l'une quelconque des revendications précédentes **caractérisée en ce que** le polymère anionique est choisi parmi le groupe des polymères anioniques à groupement sulfonique comprenant les sels de l'acide polystyrène sulfonique, les sels de métaux alcalins et alcalino terreux des acides sulfoniques dérivés de la lignine, les sels de polyacrylamide sulfoniques, les polymères contenant des motifs alkylnaphtalène sulfonique salifiés, les polymères contenant un motif vinylsulfonique, les copolymères résultant de la polymérisation d'un acide sulfonique insaturé et d'un N-monoalkylacrylamide.

8. Utilisation selon la revendication 7, **caractérisé en ce que** le polymère anionique est le produit de la copolymérisation de l'acide acrylamido-2 méthyl-2 propane sulfonique (40 à 70%) et de tertiobutylacrylamide.

9. Utilisation selon l'une quelconque des revendications 1 à 6 **caractérisée en ce que** le polymère anionique est un polymère à fonction acide carboxylique ayant la formule générale suivante : 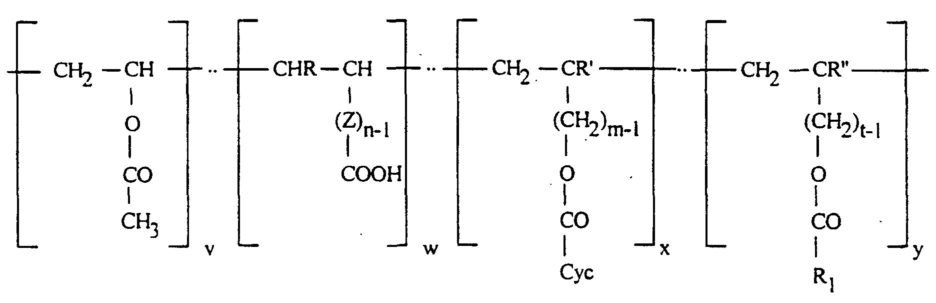 dans laquelle :
R. R' et R", identiques ou différents, représentent un atome d'hydrogène ou un radical méthyle,
m, n et t sont 1 ou 2,
R₁ représente un radical alkyle, linéaire ou ramifié, saturé ou insaturé ayant de 2 à 21 atomes de carbone.
Z représente un radical divalent pris dans le groupe constitué par : -CH₂-. - CH₂-O-CH₂- et -CH₂-O-(CH₂)₂-.
Cyc représente un radical choisi parmi :
(i) un radical de formule: 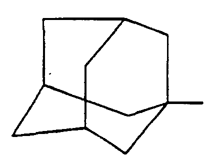
(ii) un radical de formule 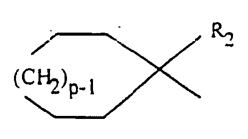 dans laquelle :
R₂ représente un atome d'hydrogène ou un radical méthyle,
et p est 1 ou 2.
(iii) un radical de formule : 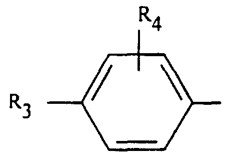 dans laquelle :
R₃ représente un atome d'hydrogène, un radical méthyle, éthyle, éthyle, tert-butyle, éthoxy, butoxy ou dodécyloxy et R₄ représente un atome d'hydrogène. un radical alkyle de 1 à 4 atomes de carbone ou un radical alkoxy de 1 à 4 atomes de carbone, et
(iv) un radical de formule : 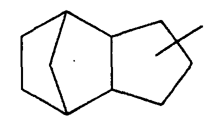
v représente de 10 à 91 % et de préférence de 36 à 84 % en poids.
w représente de 3 à 20 % et de préférence de 6 à 12 % en poids,
x représente de 4 à 60 % et de préférence de 6 à 40 % en poids,
et y représente de 0 à 40 % et de préférence de 4 à 30 % en poids,
v + w + x + y étant égal à 100 %.

10. Utilisation selon la revendication 9 **caractérisé en ce que** le polymère anionique est un terpolymère acétate de vinyle / tertio-butylbenzoate de vinyle / - acide crotonique.

11. Utilisation selon l'une quelconque des revendications 1 à 6 **caractérisée en ce que** le polymère anionique est un copolymère vinylpyrrolidone / acide acrylique ou méthacrylique comprenant éventuellement d'autres comonomères.

12. Utilisation selon l'une quelconque des revendications 1 à 6 **caractérisée en ce que** le polymère anionique est un copolymère acide méthacrylique/acrylate ou methacrylate d'alkyle.

13. Utilisation selon l'une quelconque des revendications 1 à 6 **caractérisée en ce que** le polymère anionique est un copolymère acide acrylique/N.N-diméthylacrylamide/méthacrylate d'éthyle/N-tertiobutylacrylamide.

14. Utilisation selon l'une quelconque des revendications précédentes **caractérisée en ce que** le polymère anionique est mis en oeuvre en une quantité comprise entre 0,1 et 25% en poids et de préférence comprise entre 0,5 et 20%.

15. Utilisation selon l'une quelconque des revendications précédentes **caractérisée en ce que** le copolymère bloc linéaire est utilisé en une quantité comprise entre 0,05 et 20% en poids et de préférence comprise entre 0,1 et 10%.

16. Composition cosmétique pour le maintien de la coiffure **caractérisée en ce qu'**elle contient, dans un support cosmétiquement acceptable, un copolymère bloc linéaire polysiloxane-polyoxyalkylène tel que défini à l'une quelconque des revendications 1 à 6 et un polymère anionique tel que défini à l'une quelconque des revendications 1 et 7 à 12 à l'exclusion des polymères anioniques suivants : copolymère anhydride maléique / méthyl vinyl ether monoestérifié par un alcool inférieur, copolymère acétate de vinyle / acide crotonique et copolymère acide acrylique / ester acrylique / N-alkyl acrylamide.

17. Composition selon la revendication 16 **caractérisée en ce que** le polymère anionique est mis en oeuvre en une quantité comprise entre 0,1 et 25% en poids et de préférence comprise entre 0,5 et 20%.

18. Composition selon la revendication 16 ou 17 **caractérisée en ce que** le copolymère bloc linéaire est utilisé en une quantité comprise entre 0,05 et 20% en poids et de préférence comprise entre 0,1 et 10%

## Patentansprüche

1. Verwendung eines linearen Polysiloxan-Polyoxyalkylen-Blockcopolymers als Mittel zur Erhöhung des Fixiervermögens einer Zusammensetzung für den Halt der Frisur, die ein anionisches Polymer enthält.

2. Verwendung nach Anspruch 1, **dadurch gekennzeichnet, daß** das lineare Blockcopolymer der folgenden allgemeinen Formel entspricht:
([Y(R₂SiO)ₐR'₂SiYO][(CₙH₂ₙO)_{b}])_{c}
worin bedeuten:
- die Gruppen R und R', die identisch oder voneinander verschieden sind, eine einwertige Kohlenwasserstoffgruppe, die keine aliphatische Doppelbindung aufweist,
- n eine ganze Zahl von 2 bis 4,
- a 5 oder eine ganze Zahl über 5,
- b 4 oder eine ganze Zahl über 4,
- c 4 oder eine ganze Zahl über 4,
- Y eine zweiwertige organische Gruppe, die über eine Kohlenstoff-Silicium-Bindung an das angrenzende Siliciumatom und über ein Sauerstoffatom an einen Polyoxyalkylenblock gebunden ist;
wobei:
- das mittlere Molekulargewicht jedes Siloxanblocks im Bereich von etwa 400 bis etwa 10 000 und jedes Polyoxyalkylenblocks im Bereich von etwa 300 bis etwa 10 000 liegt,
- die Siloxanblöcke etwa 10 bis etwa 90 Gew.-% des Blockcopolymers ausmachen, und
- das Zahlenmittel des Molekulargewichts des Blockcopolymers mindestens 3 000 beträgt.

3. Verwendung nach Anspruch 2, **dadurch gekennzeichnet, daß** die Gruppen R und R' unter Methyl, Ethyl, Propyl, Butyl, Pentyl, Hexyl, Octyl, Decyl, Docecyl, Phenyl, Naphthyl, Benzyl, Phenylethyl, Tolyl, Xylyl und Cyclohexyl ausgewählt sind.

4. Verwendung nach einem der Ansprüche 2 und 3, **dadurch gekennzeichnet, daß** die Gruppe Y unter -R"-, -R"-CO-, -R"-NHCO-, -R"-NH-CONH-R"'-NHCO oder -R"-OCONH-R"'-NHCO- ausgewählt ist, wobei R" Ethylen, Propylen oder Butylen und R"' -C₆H₄-, -C₆H₄-C₆H₄-, -C₆H₄-CH₂-C₆H₄- und -C₆H₄-CH(CH₃)₂-C₆H₄- bedeutet.

5. Verwendung nach einem der vorhergehenden Ansprüche, **dadurch gekennzeichnet, daß** das Blockcopolymer unter den Verbindungen der folgenden Formel ausgewählt ist:
[C₄H₈(CₙH₂ₙO)_{b}- C₄H₈ -SiMe₂O(SiMe₂O)ₐSiMe₂]_{c}
worin bedeuten: Me Methyl, n eine ganze Zahl von 2 bis 4, a und b 4 oder eine ganze Zahl über 4, und c 4 oder eine Zahl über 4.

6. Verwendung nach einem der vorhergehenden Ansprüche, **dadurch gekennzeichnet, daß** das Blockcopolymer unter den folgenden Verbindungen ausgewählt ist:
[[(CH₃)₂SiO]₄₁(CH₃)₂SiCH₂CH(CH₃)CH₂-O(C₂H₄O)₁₈-(C₃H₆O)₃₃-CH₂CH(CH₃)CH₂]_{16.1}
[[(CH₃)₂SiO]₃₁(CH₃)₂SiCH₂CH(CH₃)CH₂-O(C₂H₄O)₂₀-(C₃H₆O)₂₉-CH₂CH(CH₃)CH₂]_{13.3}
[[(CH₃)₂SiO]₉(CH₃)₂SiCH₂CH(CH₃)CH₂-O(C₂H₄O)₂₀-(C₃H₆O)₂₉-CH₂CH(CH₃)CH₂]_{26.3}
[[(CH₃)₂SiO]₁₆(CH₃)₂SiCH₂CH(CH₃)CH₂-O(C₂H₄O)₁₈-(C₃H₆O)₂₀-CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8},

7. Verwendung nach einem der vorhergehenden Ansprüche, **dadurch gekennzeichnet, daß** das anionische Polymer aus der Gruppe der anionischen Polymere mit Sulfonsäuregruppe ausgewählt ist, die die Salze der Polystyrolsulfonsäure, die Alkalimetall- und Erdalkalimetallsalze der von Lignin abgeleiteten Sulfonsäuren, die Salze der Polyacrylamidsulfonsäuren, die Polymere, die Alkylnaphthalinsulfonsäureeinheiten in Salzform enthalten, die Polymere mit Vinylsulfonsäureeinheiten und die Copolymere umfaßt, die bei der Polymerisation einer ungesättigten Sulfonsäure und N-Monoalkylacrylamid entstehen.

8. Verwendung nach Anspruch 7, **dadurch gekennzeichnet, daß** das anionische Polymer das Produkt der Coplymerisation von 2-Acrylamido-2-methyl-propansulfonsäure (40 bis 70 %) und t-Butylacrylamid ist.

9. Verwendung nach einem der Ansprüche 1 bis 6, **dadurch gekennzeichnet, daß** das anionische Polymer ein Polymer mit Carbonsäuregruppe ist, das die folgende allgemeine Formel aufweist: 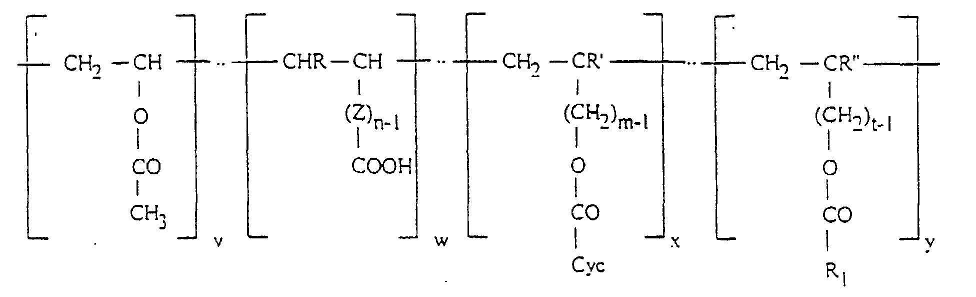 worin bedeuten:
die Gruppen R, R' und R", die identisch oder voneinander verschieden sind, Wasserstoff oder Methyl,
m, n und t 1 oder 2,
R₁ eine geradkettige oder verzweigte, gesättigte oder ungesättigte Alkylgruppe mit 2 bis 21 Kohlenstoffatomen,
Z eine zweiwertige Gruppe, die unter -CH₂-, -CH₂-O-CH₂ und
CH₂-O-(CH₂)₂- ausgewählt ist,
Cyc eine Gruppe, die ausgewählt ist unter:
(i) der Gruppe der Formel: 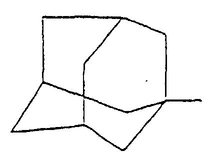
(ii) einer Gruppe der Formel: 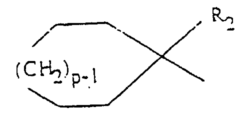 worin bedeuten:
R₂ Wasserstoff oder Methyl, und
p 1 oder 2;
(iii) einer Gruppe der Formel: 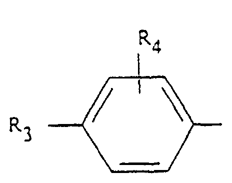 worin bedeuten:
R₃ Wasserstoff, Methyl, Ethyl, t-Butyl, Ethoxyy, Butoxy oder Dodecyloxy, und R₃ Wasserstoff, eine Alkygruppe mit 1 bis 4 Kohlenstoffatomen oder eine Alkygruppe mit 1 bis 4 Kohlenstoffatomen;
(iv) der Gruppe der Formel: 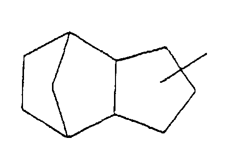
v 10 bis 91 % und vorzugsweise 36 bis 84 Gew.-%,
w 3 bis 20 % und vorzugsweise 6 bis 12 Gew.-%,
x 4 bis 60 % und vorzugsweise 6 bis 40 Gew.-%,
y 0 bis 40 % und vorzugsweise 4 bis 30 Gew.-%,
wobei v + w + x + y 100 % ausmachen.

10. Verwendung nach Anspruch 9, **dadurch gekennzeichnet, daß** das anionische Polymer ein Vinylacetat/Vinyl-t-butylbenzoat/Crotonsäure-Terpolymer ist.

11. Verwendung nach einem der Ansprüche 1 bis 6, **dadurch gekennzeichnet, daß** das anionische Polymer ein Copolymer von Vinylpyrrolidon/Acrylsäure oder Methacrylsäure ist, das gegebenenfalls weitere Comonomere enthält.

12. Verwendung nach einem der Ansprüche 1 bis 6, **dadurch gekennzeichnet, daß** das anionische Polymer ein Copolymer von Methacrylsäure/Alkylacrylat oder Alkylmethacrylat ist.

13. Verwendung nach einem der Ansprüche 1 bis 6, **dadurch gekennzeichnet, daß** das anionische Polymer von Acrylsäure/N,N-Dimethylacrylamid/Ethylmethacrylat/N-t-Butylacrylamid-Copolymer ist.

14. Verwendung nach einem der vorhergehenden Ansprüche, **dadurch gekennzeichnet, daß** das anionische Polymer in einer Menge von 0,1 bis 25 Gew.-% und vorzugsweise 0,5 bis 20 % verwendet wird.

15. Verwendung nach einem der vorhergehenden Ansprüche, **dadurch gekennzeichnet, daß** das lineare Blockcopolymer in einem Mengenanteil von 0,05 bis 20 Gew.-% und vorzugsweise 0,1 bis 10 Gew.-% verwendet wird.

16. Kosmetische Zusammensetzung für den Halt der Frisur, **dadurch gekennzeichnet, daß** sie in einem kosmetisch akzeptablen Träger ein lineares Polysiloxan-Polyoxyalkylen-Blockcopolymer nach einem der Ansprüche 1 bis 6 und ein anionisches Polymer nach einem der Ansprüche 1 und 7 bis 12 enthält, wobei die folgenden anionische Polymere ausgenommen sind: Copolymer Maleinsäureanhydrid/ einfach mit einem niederen Alkohol veresterter Methylvinylether, Copolymer Vinylacetat/Crotonsäure und Copolymer Acrylsäure/Acrylester / N-Alkylacrylamid.

17. Zusammensetzung nach Anspruch 16, **dadurch gekennzeichnet, daß** das anionische Polymer in einer Menge von 0,1 bis 25 Gew.-% und vorzugsweise 0,5 bis 20 % verwendet wird.

18. Zusammensetzung nach Anspruch 16 oder 17, **dadurch gekennzeichnet, daß** das lineare Blockcopolymer in einer Menge von 0,05 bis 20 Gew.-% und vorzugsweise 0,1 bis 10 % verwendet wird.

## Claims

1. Use of a polysiloxane/polyoxyalkylene linear 'block copolymer as an agent for enhancing the fixing power of a composition for maintaining hairstyle containing an anionic polymer.

2. Use according to Claim 1, **characterized in that** the linear block copolymer corresponds to the general formula
( [Y(R₂SiO)ₐR'₂SiYO][(CₙH₂ₙO)_{b}])_{c}
in which:
- R and R', which may be identical or different, represent a monovalent hydrocarbon radical not containing an aliphatic unsaturation,
- n is an integer between 2 and 4,
- a is an integer greater than or equal to 5,
- b is an integer greater than or equal to 4,
- c is an integer greater than or equal to 4,
- Y represents a divalent organic group which is linked to the adjacent silicon atom via a carbon-silicon bond and to a polyoxyalkylene block via an oxygen atom,
- the average molecular weight of each siloxane block is between approximately 400 and approximately 10,000, that of each polyoxyalkylene block being between approximately 300 and approximately 10,000,
- the siloxane blocks represent from approximately 10% to approximately 90% by weight of the block copolymer,
- the average molecular weight of the block copolymer being at least 3,000.

3. Use according to Claim 2, **characterized in that** R and R' are chosen from the group comprising methyl, ethyl, propyl, butyl, pentyl, hexyl, octyl, decyl, dodecyl, phenyl, naphthyl, benzyl, phenylethyl, tolyl, xylyl and cyclohexyl radicals.

4. Use according to either of Claims 2 and 3, **characterized in that** Y is chosen from the group comprising -R"-, -R"-CO-, -R"-NHCO-, -R"-NH-CONH-R"'-NHCO or -R"-OCONH-R"'-NHCO-, where R" represents an ethylene, propylene or butylene radical and R"' represents a -C₆H₄-, -C₆H₄-C₆H₄-, -C₆H₄-CH₂-C₆H₄- or -C₆H₄-CH(CH₃)₂-C₆H₄-group.

5. Use according to any one of the preceding claims, **characterized in that** the block copolymer is chosen from those of formula:
[C₄H₈(CₙH₂ₙO)_{b}-C₄H₈-SiMe₂O (SiMe₂O)ₐSiMe₂]_{c}
where Me represents methyl, n is an integer from 2 to 4, a and b are integers greater than or equal to 4 and c is a number greater than or equal to 4.

6. Use according to any one of the preceding claims, **characterized in that** the block copolymer is chosen from the group comprising the following compounds:
[[(CH₃)₂SiO]₄₁(CH₃)₂SiCH₂CH(CH₃)CH₂-O(C₂H₄O)₁₈(C₃H₆O)₃₃CH₂CH(CH₃)CH₂]_{16.1}
[[(CH₃)₂SiO]₃₁(CH₃)₂SiCH₂CH(CH₃)CH₂-O(C₂H₄O)₂₀(C₃H₆O)₂₉CH₂CH(CH₃)CH₂]_{13.3}
[[(CH₃)₂SiO]₉(CH₃)₂SiCH₂CH(CH₃)CH₂-O(C₂H₄O)₂₀(C₃H₆O)₂₉CH₂CH(CH₃)CH₂]_{26.3}
[[(CH₃)₂SiO]₁₆(CH₃)₂SiCH₂CH(CH₃)CH₂-O(C₂H₄O)₁₈(C₃H₆)₂₀CH₂CH(CH₃)CH₂]_{21.5}
[[(CH₃)₂SiO]₉(CH₃)₂SiCH₂CH(CH₃)CH₂-O(C₂H₄O)₅-CH₂CH(CH₃)CH₂]_{4.8}

7. Use according to any one of the preceding claims, **characterized in that** the anionic polymer is chosen from the group of anionic polymers containing a sulphonic group, comprising polystyrenesulphonic acid salts, alkali metal and alkaline-earth metal salts of sulphonic acids derived from lignin, polyacrylamide-sulphonic acid salts, polymers containing salified alkylnaphthalenesulphonic units, polymers containing a vinylsulphonic unit and copolymers resulting from the polymerization of an unsaturated sulphonic acid and an N-monoalkylacrylamide.

8. Use according to Claim 7, **characterized in that** the anionic polymer is the copolymerization product of 2-acrylamido-2-methylpropanesulphonic acid (40 to 70%) and tert-butylacrylamide.

9. Use according to any one of Claims 1 to 6, **characterized in that** the anionic polymer is a polymer containing a carboxylic acid function having the following general formula: 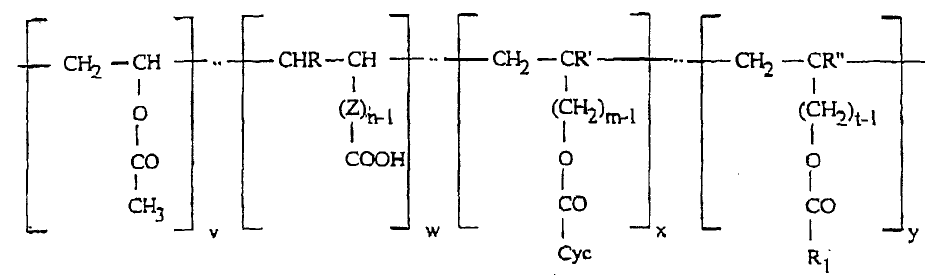 in which:
R, RN and RO, which may be identical or different, represent a hydrogen atom or a methyl radical,
m, n and t are 1 or 2,
R₁ represents a saturated or unsaturated, linear or branched alkyl radical having from 2 to 21 carbon atoms,
Z represents a divalent radical taken from the group consisting of: -CH₂-, -CH₂-O-CH₂- and -CH₂-O- (CH₂)₂-,
Cyc represents a radical chosen from:
(i) a radical of formula: 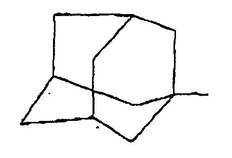
(ii) a radical of formula: 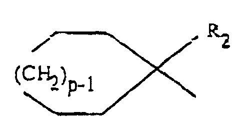 in which:
R₂ represents a hydrogen atom or a methyl radical, and p is 1 or 2,
(iii) a radical of formula: 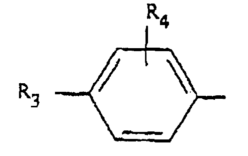 in which:
R₃ represents a hydrogen atom or a methyl, ethyl, text-butyl, ethoxy, butoxy or dodecyloxy radical and R₄ represents a hydrogen atom, an alkyl radical having 1 to 4 carbon atoms or an alkoxy radical having 1 to 4 carbon atoms,
and (iv) a radical of formula: 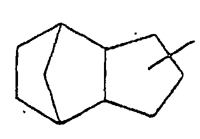
v represents from 10 to 91% and preferably from 36 to 84% by weight,
w represents from 3 to 20% and preferably from 6 to 12% by weight,
x represents from 4 to 60% and preferably from 6 to 40% by weight,
and y represents from 0 to 40% and preferably from 4 to 30% by weight,
v + w + x + y being equal to 100%.

10. Use according to Claim 9, **characterized in that** the anionic polymer is a vinyl acetate/vinyl tert-butyl-benzoate/crotonic acid terpolymer.

11. Use according to any one of Claims 1 to 6, **characterized in that** the anionic polymer is a vinyl-pyrrolidone/acrylic or methacrylic acid copolymer optionally comprising other comonomers.

12. Use according to any one of Claims 1 to 6, **characterized in that** the anionic polymer is a methacrylic acid/alkyl acrylate or methacrylate copolymer.

13. Use according to any one of Claims 1 to 6, **characterized in that** the anionic polymer is an acrylic acid/N,N-dimethylacrylamide/ethyl methacrylate/N-tert-butylacrylamide copolymer.

14. Use according to any one of the preceding claims, **characterized in that** the anionic polymer is employed in an amount between 0.1 and 25% by weight, and preferably between 0.5 and 20%.

15. Use according to any one of the preceding claims, **characterized in that** the linear block copolymer is used in an amount between 0.05 and 20% by weight, and preferably between 0.1 and 10%.

16. Cosmetic composition for maintaining hairstyle, **characterized in that** it contains, in a cosmetically acceptable carrier, a polysiloxane/polyoxyalkylene linear block copolymer as defined in any one of Claims 1 to 6 and an anionic polymer as defined in any one of Claims 1 and 7 to 12 excluding the following anionic polymers: maleic anhydride/methyl vinyl ether copolymer monoesterified with a lower alcohol, vinyl acetate/crotonic acid copolymer and acrylic acid/acrylic ester/N-alkylacrylamide copolymer.

17. Composition according to Claim 16, **characterized in that** the anionic polymer is employed in an amount between 0.1 and 25% by weight, and preferably between 0.5 and 20%.

18. Composition according to Claim 16 or 17, **characterized in that** the linear block copolymer is used in an amount between 0.05 and 20% by weight, and preferably between 0.1 and 10%.
